(19) **Europäisches Patentamt**

**European Patent Office**

**Office européen des brevets**

(11) **EP 0 723 017 A2**

(12) **EUROPÄISCHE PATENTANMELDUNG**

(43) Veröffentlichungstag:
24.07.1996 Patentblatt 1996/30

(51) Int. Cl.⁶: **C12N 15/54**, C12N 9/10,
C12Q 1/48, A01N 3/00,
C12N 1/21, C12N 5/10

(21) Anmeldenummer: 96100458.7

(22) Anmeldetag: 13.01.1996

(84) Benannte Vertragsstaaten:
**AT BE CH DE DK ES FR GB IT LI NL**

(30) Priorität: 23.01.1995 DE 19501906

(71) Anmelder: **BASF AKTIENGESELLSCHAFT**
**67056 Ludwigshafen (DE)**

(72) Erfinder:
- **Schmidt, Ralf-Michael, Dr.**
  **D-67434 Neustadt (DE)**
- **Stitt, Marc, Prof. Dr.**
  **D-69221 Dossenheim (DE)**
- **Sonnewald, Uwe, Dr.**
  **D-06467 Hoym (DE)**

(54) **Transketolase**

(57) Protein mit Transketolase Aktivität, enthaltend eine Aminosäuresequenz, die eine Teilsequenz von mindestens 100 Aminosäuren aus SEQ ID NO 2 darstellt, sowie für dieses Protein kodierende Nukleinsäuren und seine Verwendung.

EP 0 723 017 A2

**Beschreibung**

Die vorliegende Erfindung betrifft Proteine mit Transketolase Aktivität, ihre Verwendung in Testsystemen, sowie Nukleinsäuren, die für diese Proteine codieren.

Pflanzen sind in der Lage, unter Verwendung von Lichtenergie aus atmosphärischem Kohlendioxid organische Verbindungen unter Sauerstoffbildung aufzubauen. Dieser Vorgang wird als Photosynthese bezeichnet.

Es ist anzunehmen, daß die effiziente Bildung, Nutzung und Verteilung der Photosyntheseprodukte das Wachstum einer Pflanze stark beeinflussen.

Da Pflanzen auf eine funktionierende Photosynthese angewiesen sind und vergleichbare Reaktionen in tierischen Organismen nicht vorkommen, bietet sich der Photosyntheseapparat als ideales Ziel für den Einsatz von Herbiziden an.

Die komplexen Reaktionen, die zur Kohlendioxidfixierung führen unterteilt man in Licht- und Dunkelreaktion. Die Lichtreaktion dient der Bereitstellung von Energie in Form von ATP und von Reduktionsäquivalenten in Form von NADPH. In der Dunkelreaktion (reduktiver Pentosephosphatzyklus oder Calvin Zyklus) werden diese Verbindungen zur Synthese organischer Kohlenstoffverbindungen genutzt.

Einige der bekannten Herbizide (z.B. Dichlorphenylmethylharnstoff oder Paraquat) wirken durch eine Inhibierung der Lichtreaktion. Die Dunkelreaktion wird als Angriffspunkt für Herbizide nicht genutzt.

Die Enzymreaktionen des reduktiven Pentosephosphatzyklus werden in drei Abschnitte unterteilt:

a) Carboxylierung
b) Reduktion
c) Regenerierung.

Bei der Carboxylierung reagiert Kohlendioxid mit dem Akzeptormolekül Ribulose-Bisphosphat (RuBP), wodurch zwei Moleküle 3-Phosphoglycerat (3-PGA) gebildet werden. Anschließend wird 3-PGA nach Phosphorylierung zu Glycerinaldehyd-3-Phosphat (GAP) reduziert. In der Regenerationsphase wird das Akzeptormolekül RuBP aus dem entstandenen GAP resynthetisiert. Von sechs gebildeten Molekülen GAP kann ein Molekül für andere Stoffwechselwege eingesetzt werden.

Eine Vielzahl der am reduktiven Pentosephosphatzyklus beteiligten Enzyme stellen potentielle Angriffspunkte für Herbizide dar. Eine besondere Stellung nimmt allerdings die plastidäre Transketolase ein. Wie die Transaldolase katalysiert die Transketolase (E.C. 2.2.1.1.) zwei Reaktionen:

(1)

Fruktose-6-Phosphat + Glycerinaldehyd-3-Phosphat --> Erythrose-4-Phosphat + Xylulose-5-Phosphat

(2)

Sedoheptulose-7-Phosphat + Glycerinaldehyd-3-Phosphat --> Ribose-5-Phosphat + Xylulose-5-Phosphat

Die an den Reaktionen beteiligten Substrate und Produkte stellen Verknüpfungspunkte des reduktiven Pentosephosphatzykluses mit anderen Stoffwechselwegen dar. Exportierte Triosephosphate dienen im Zytoplasma als Substrate für Glykolyse und Gluconeogenese. Fruktose-6-Phosphat wird als Vorläufermolekül zur Herstellung von Stärke in den Plastiden genutzt. Erythrose-4-Phosphat ist ein Mittler zwischen Primär- und Sekundärstoffwechsel. Verknüpft mit Phosphoenolpyruvat mündet Erythrose-4-Phosphat in den Shikimat-Weg, der zur Synthese aromatischer Aminosäuren und phenolischer Substanzen führt.

Ribose-5-Phosphat wird in unterschiedlichen Stoffwechselwegen als Substrat verwendet.

In pflanzlichen Geweben wurden zwei Transketolase-Isoformen beschrieben, die sich in ihrer subzellulären Kompartimentierung unterscheiden (Murphy and Walker, 1982, Planta 155, 316-320).

Die plastidäre Transketolase ist in grünen Geweben für mehr als 75% der Gesamtaktivität verantwortlich. Das aktive Enzym liegt als Homotetramer (Holoenzym) mit einer relativen Molekularmasse von 150 kDa vor. Als Cofaktoren benötigt die Transketolase Vitamin B1 (Thiaminpyrophosphat) und Magnesium. In Abwesenheit von Thiaminpyrophosphat oder in Anwesenheit von Mercaptoethanol zerfällt das Tetramer in zwei Dimere (Apoenzyme) mit einer relativen Molekularmasse von je 74 kDa. Holo- und Apoenzym sind katalytisch aktiv, wobei das Holoenzym eine wesentlich höhere Aktivität als das Apoenzym aufweist.

Gene, die für Transketolase kodieren, wurden bisher aus Saccharomyces cerevisiae (Flechter et al., Biochemistry 31, 1892-1896, 1993; Sundström et al., J. Biol. Chem. 268, 24346-24352, 1993; Schaff-Gerstenschläger et al., Eur. J. Biochem. 217, 487-492, 1993), aus Hansenula polymorpha (Janowicz et al., Nucl. Acids Res. 13, 3043-3062, 1985), menschlichen Erythrozyten (Abedinia et al., Biochem. Biophys. Res. Commun. 183, 1159-1166, 1992; McCool et al., J. Biol. Chem. 268, 1397-1404, 1993), Rhodobacter sphaeroides (Chen et al., J. Biol. Chem. 266, 20447-20452, 1992) und Escherichia coli (Sprenger, Biochem. Biophys. Acta 1216, 307-310, 1992; Tida et al., J. Bacteriol. 175, 5375-5383, 1993) isoliert und beschrieben. Gene pflanzlicher Transketolasen sind bisher nicht bekannt.

Aufgabe der vorliegenden Erfindung war es, eine pflanzliche Transketolase in reiner Form durch Klonierung des entsprechenden Gens zur Verfügung zu stellen.

Demgemäß wurde ein Protein mit Transketolase Aktivität, enthaltend die in SEQ ID NO 2 dargestellte Aminosäuresequenz, gefunden.

Die in SEQ ID NO 2 dargestellte Aminosäuresequenz beruht auf der Translation der in SEQ ID NO 1 dargestellten cDNA Sequenz.

Das in SEQ ID NO 2 dargestellte Protein ist ein sogenanntes Vorläuferprotein bestehend aus 743 Aminosäuren. Das reife Protein ist aus der Vorläuferform durch Abspalten des chloroplastidären Transitpeptides, das gemäß einer Computerananlyse aus den N-terminalen 77 Aminosäuren besteht, erhältlich.

Sowohl das Vorläuferprotein, als auch durch Substitution, Deletion oder Insertion von Aminosäuren davon abgeleitete Proteine, die noch über eine Transketolase-Aktivität verfügen, gehören zu den erfindungsgemäßen Proteinen.

Unter Substitution ist der Austausch einer oder mehrerer Aminosäuren durch eine oder mehrere andere Aminosäuren zu verstehen. Bevorzugt werden sog. konservative Austausche durchgeführt, bei denen die ersetzte Aminosäure eine ähnliche Eigenschaft hat wie die ursprüngliche Aminosäure, beispielsweise Austausch von Glu durch Asp, Val durch Ile, Ser durch Thr.

Deletion ist das Ersetzen einer Aminosäure durch eine direkte Bindung; bevorzugte Positionen für Deletionen sind die Termini des Polypeptides und die Verknüpfungen zwischen den einzelnen Proteindomänen.

Insertionen sind Einfügungen von Aminosäuren in die Polypeptidkette, wobei formal eine direkte Bindung durch eine oder mehrere Aminosäuren ersetzt wird.

Besonders bevorzugt sind Proteine, die durch N-terminale Verkürzungen um 20 bis 100 Aminosäuren aus SEQ ID NO 2 entstehen.

Ein weiterer Gegenstand der Erfindung sind Nukleinsäuren, die für die oben genannten Proteine kodieren. Geeignete Nukleinsäuresequenzen sind durch Rückübersetzung der Polypeptidsequenz gemäß dem genetischen Code erhältlich. Bevorzugt werden dafür solche Codons verwendet, die entsprechend der Organismus spezifischen codon usage häufig verwendet werden. Die codon usage läßt sich anhand von Computerauswertungen anderer, bekannter Gene des betreffenden Organismus leicht ermitteln.

Soll die pflanzliche Transketolase beispielsweise in einem Bakterium exprimiert werden, so ist es häufig vorteilhaft, die codon usage des Bakteriums bei der Rückübersetzung zu verwenden.

Ein weiterer Gegenstand der Erfindung sind Vektoren, die die für die erfindungsgemäße Transketolase kodierenden Nukleinsäuren zusammen mit funktionellen Regulationssignalen enthalten.

Darunter sind beispielsweise Signale für Transkription und Translation wie Promotoren und Ribosomenbindungsstellen oder für Replikation oder Integration notwendige Sequenzen zu verstehen.

Die erfindungsgemäßen Proteine eignen sich besonders zur Identifizierung von herbiziden Wirkstoffen, insbesondere zur Auffindung von Transketolase spezifischen Hemmstoffen.

Dazu können die Proteine beispielsweise in einem Enzymtest eingesetzt werden, bei dem die Aktivität der Transketolase in An- und Abwesenheit des zu testenden Wirkstoffs ermittelt wird. Aus dem Vergleich der beiden Aktivitätsbestimmungen läßt sich eine qualitative und quantitative Aussage über das Hemmverhalten des zu testenden Wirkstoffes machen.

Mit Hilfe des erfindungsgemäßen Testsystems kann eine Vielzahl von chemischen Verbindungen schnell und einfach auf herbizide Eigenschaften überprüft werden.

Ein weiterer Gegenstand der Erfindung sind Herbizide, die mit einem oben beschriebenen Testsystem identifizierbar sind.

Die Erfindung besteht außerdem in einem Verfahren zur Herstellung von Herbiziden, die eine pflanzliche Transketolase inhibieren, das dadurch gekennzeichnet ist, daß man bekannte chemische Verbindungen in einem oben beschriebenen Testverfahren überprüft und solche mit inhibierender Wirkung mit üblichen Träger- und Hilfsstoffen als Herbizid formuliert.

Daß die Transketolase inhibierende Eigenschaft einer Substanz allein nicht ausreicht für die Eignung als Herbizid, sondern noch weitere Prüfungen durchzuführen sind, ist jedem Fachmann geläufig.

Das Verfahren gestattet es jedoch reproduzierbar aus einer großen Anzahl von Substanzen gezielt solche mit großer Wirkstärke auszuwählen, um mit diesen Substanzen anschließend weitere, dem Fachmann geläufige vertiefte Prüfungen durchzuführen.

Die Erfindung wird durch die folgenden Beispiele weiter veranschaulicht.

Beispiele

A. Gentechnische Verfahren, die den Ausführungsbeispielen B zugrundeliegen:

1. Allgemeine Klonierungsverfahren

Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von E. coli Zellen, Anzucht von Bakterien, Vermehrung von Phagen und Sequenzanalyse rekombinanter DNA wurden wie bei Sambrook et al. (1989) (Cold Spring Harbor Laboratory Press: ISBN 0-87969-309-6) beschrieben durchgeführt. Transformation und Anzucht von Pichia pastoris wurde entsprechend den Angaben der Vertreiberfirma (Invitrogen Corporation) durchgeführt. Die Transformation von Agrobacterium tumefaciens wurde entsprechend der Methode von Höfgen und Willmitzer (Nucl. Acid Res. (1988) 16, 9877) ausgeführt. Die Anzucht der Agrobakterien erfolgte in YEB Medium (Vervliet et al. J. Gen. Virol. (1975) 26, 33).

2. Erzeugung von cDNA-Bibliotheken

Zur Herstellung von Blatt-spezifischen cDNA Bibliotheken wurde Gesamt-RNA aus Tabakblättern nach einer von Logemann et al. (Anal. Biochem. (1987) 163,21) beschriebenen Methode isoliert. Anschließend wurde die poly(A)-RNA über Oligo(dT)-Cellulose Type 7 (Pharmacia, Freiburg) nach Angaben des Herstellers gereinigt. Nach photometrischer Konzentrationsbestimmung wurden 5 μg der so erhaltenen RNA für die cDNA Synthese eingesetzt. Alle für die Herstellung der cDNA notwendigen Chemikalien und Enzyme wurden durch die Firma Stratagene (La Jolla CA 92037, USA) bezogen. Die angewandten Methoden wurden nach Angaben des Herstellers durchgeführt. Die Synthese des ersten und zweiten Stranges der cDNA wurde mit dem ZAP-cDNA Synthese Kit durchgeführt. Die erhaltenen doppelsträngigen cDNAs wurden anschließend mit EcoRI-NotI Adaptoren versehen und in einen EcoRI gespaltenen Lambda ZAPII Vektor kloniert. Nach in vitro Verpackung (Gigapack II Verpackungsextrakt) der rekombinanten Lambda DNA wurden XL-1 E. coli Zellen (Stratagene) transformiert. Durch Auszählen der gebildeten Plaques wurde der Titer der cDNA-Bibliotheken bestimmt.

3. Durchmusterung einer cDNA-Bibliothek mittels heterologer DNA-Sonden

$2 \times 10^5$ rekombinante Lambda Phagen (Lambda ZapII) einer blattspezifische cDNA-Bibliothek aus Tabak (Varietät Samsun NN) wurden auf Agarplatten ausplattiert. Die Phagen-DNA wurde mittels Standardverfahren (Sambrook et al. (1989); Cold Spring Harbor Laboratory Press: ISBN 0-87969-309-6) auf Nylon-Membranen (Hybond N, Amersham Buchler) transferiert und durch Inkubation für 2 Stunden bei 80°C auf den Filtern fixiert. Als Hybridisierungssonden dienten DNA-Fragmente, die mit Hilfe eines "Multiprime DNA labelling systems" (Amersham Buchler) in Anwesenheit von $\alpha$-$^{32}$P-dCTP (spezifische Aktivität 3000 Ci/mmol) nach Herstellerangaben radioaktiv markiert wurden. Hybridisierung der Membran erfolgte nach Prähybridisierung bei 42°C in PEG-Puffer (Amasino (1986) Anal. Biochem. 152, 304-307) für 12-16 Stunden. Anschließend wurden die Filter 3 x 20 Minuten in 2 x SSC, 0,1 % SDS bei 42°C gewaschen. Positiv hybridisierende Phagen wurden durch Autoradiographie sichtbar gemacht und durch Standardtechniken gereinigt.

4. Sequenzanalyse rekombinanter DNA

Die Sequenzierung rekombinanter DNA-Moleküle erfolgte mit einem automatischen Laserfluoreszenz-DNA-Sequenzierer (A.L.F.) der Firma Pharmacia unter Verwendung Fluoreszenz-markierter Oligonukleotide nach der Methode von Sanger (Sanger et al. (1977) Proc. Natl. Acad. Sci. USA 74, 5463-5467).

5. Bakterienstämme und Hefestämme

E. coli (XL-1 Blue) Bakterien wurden von der Firma Stratagene bezogen. Der zur Pflanzentransformation eingesetzte Agrobacterium tumefaciens Stamm (C58CI mit dem Plasmid pGV 3850kan) wurde von Debleare et al. (1985, Nucl. Acid Res. 13, 4777) beschrieben. Pichia pastoris Stamm GS115 wurde von der Firma Invitrogen Corporation (San Diego, CA 92121, USA) bezogen.

6. Tabaktransformation

Zur Transformation von Tabakpflanzen (Nicotiana tabacum L. cv. Samsun NN) wurden 10 ml einer unter Selektion gewachsenen Übernachtkultur von Agrobacterium tumefaciens abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1 cm) in dieser Bakterienlösung gebadet. Anschließend wurden die Blattscheiben in Petrischalen auf MS-Medium (Murashige und Skoog, Physiol. Plant. (1962) 15,473) mit 2 % Saccharose und 0,8 % Bacto-Agar ausgelegt. Nach 2-tägiger Inkubation im Dunkeln bei 25°C wurden sie auf MS-Medium mit 100 mg/l Kanamycin, 500 mg/l Claforan, 1 mg/ml Benzylaminopurin (BAP), 0,2 mg/l Naphthylessigsäure (NAA), 1,6 % Glukose und 0,8 % Bacto-Agar übertragen und die Kultivierung (16 Stunden Licht/8 Stunden Dunkelheit) fortgesetzt. Wachsende Sprosse wurden auf hormonfreies MS-Medium mit 2 % Saccharose, 250 mg/l Claforan und 0,8 % Bacto-Agar überführt.

7. Analyse von Gesamt-RNA aus pflanzlichen Geweben

Gesamt RNA aus pflanzlichen Geweben wurde wie bei Logemann et al. (Anal. Biochem. (1987) 163,21) beschrieben isoliert. Für die Analyse wurden jeweils 20-40 µg RNA in einem Formaldehyd-haltigen 1,5 %igen Agarosegel aufgetrennt. Nach elektrophoretischer Auftrennung der RNA Moleküle wurde die RNA mittels Kapillartransfer auf eine Nylon Membran übertragen. Der Nachweis spezifischer Transkripte wurde wie bei Amasino (Anal. Biochem. (1986) 152, 304) beschrieben durchgeführt. Die als Sonde eingesetzten cDNA-Fragmente wurden mit einem Random Primed DNA Labeling Kit (Boehringer, Mannheim) radioaktiv markiert.

8. PCR-Amplifikation von Nukleinsäuren

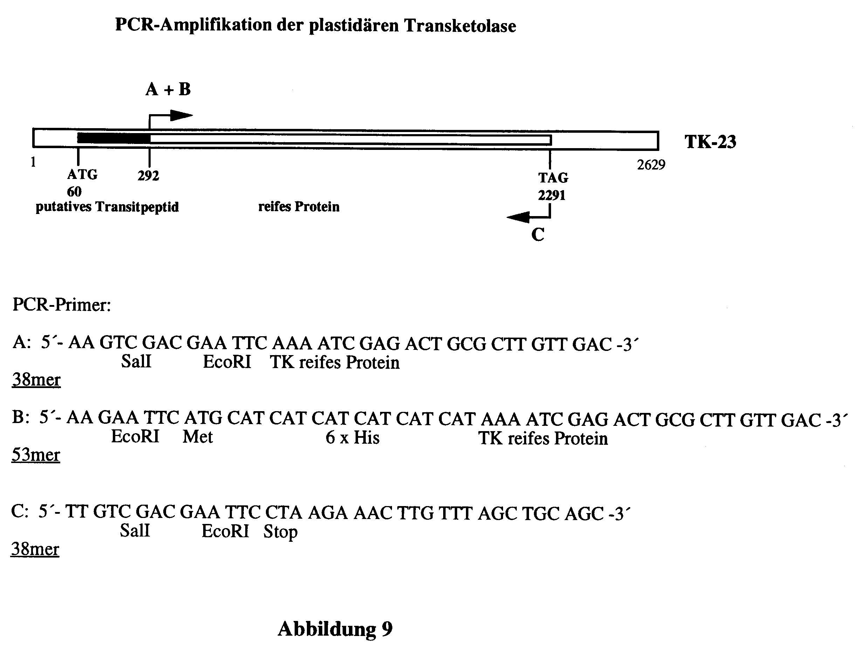

Die PCR-Amplifikation der Transketolase zur Expression des Enzyms in E. coli und Pichia wurde in einem DNA-Thermal Cycler der Firma Perkin Elmer durchgeführt. Die verwendeten Oligonukleotide sind in Abbildung 9 dargestellt. Die Reaktionsgemische enthielten 1 ng Template, 0,5 µM der entsprechenden Oligonukleotide, 0,25 mM Nukleotide (Pharmacia), Amplifikationspuffer (16 mM $(NH_4)_2SO_4$, 67 mM Tris-HCl (pH 8,8 bei 25°C), 0,01 % Tween 20, 7,5 mM $MgCl_2$) und 2,5 Einheiten der Tth DNA Polymerase (Biomaster, Crottorfer Str. 25, 51109 Köln). Die Amplifikationsbedingungen wurden wie folgt eingestellt:

Anlagerungstemperatur: 60°C
Denaturierungstemperatur: 94°C
Elongationstemperatur: 72°C
Anzahl der Zyklen: 30

9. Überexpression von Proteinen in E. coli

Zur Überexpression der Transketolase in E. coli wurden 2 ml einer bei 28°C angezogenen Übernachtkultur in 20 ml Wachstumsmedium (LB-Medium komplettiert mit 10 µg/ml Tetracyclin, 200 µg/ml Ampicillin, 1 mM Vitamin B1, 1 mM $MgSO_4$) überführt. Das Wachstum erfolgte bei 28°C unter Schütteln. Nach 3 Stunden wurde die Expression der Transketolase durch Zugabe von 2 mM IPTG induziert. Der Nachweis des produzierten Proteins wurde durch Auftrennung der Proteine in einem SDS-PAAG (Laemmli (1970) Nature 227, 680-685) mit anschließender Coomassie-Färbung der Proteine durchgeführt.

B. Ausführungsbeispiele

1. Klonierung der plastidären Transketolase

Aus einer blattspezifischen cDNA-Bibliothek aus Tabak (Varietät Samsun NN) wurde ein Klon, der für Transketolase kodiert, ausgewählt. Die DNA Sequenz ist in SEQ ID NO 1 dargestellt.

Der 2629 Basenpaar lange cDNA-Klon 21 enthält einen offenen Leseraster von 2229 Basen und kodiert für ein Protein mit 743 Aminosäuren. Analyse des Polypeptides unter Verwendung des Sequenzprogramms PC/Gene (Untermenü TRANSPEP) ergab, daß am N-Terminus des Proteins ein chloroplastidäres Transitpeptid von vermutlich 77 Aminosäuren vorhanden ist.

2. Vergleich der plastidären Transketolase aus Tabak mit bekannten Transketolase Proteinsequenzen

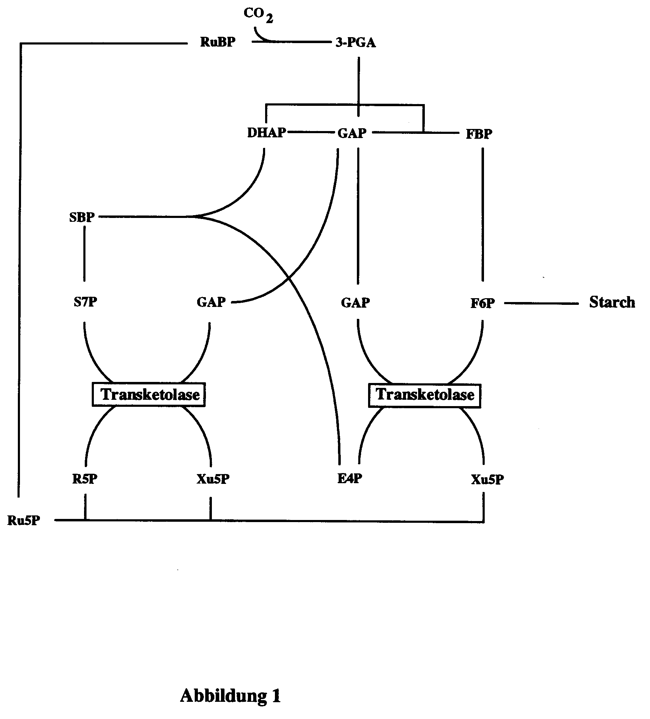

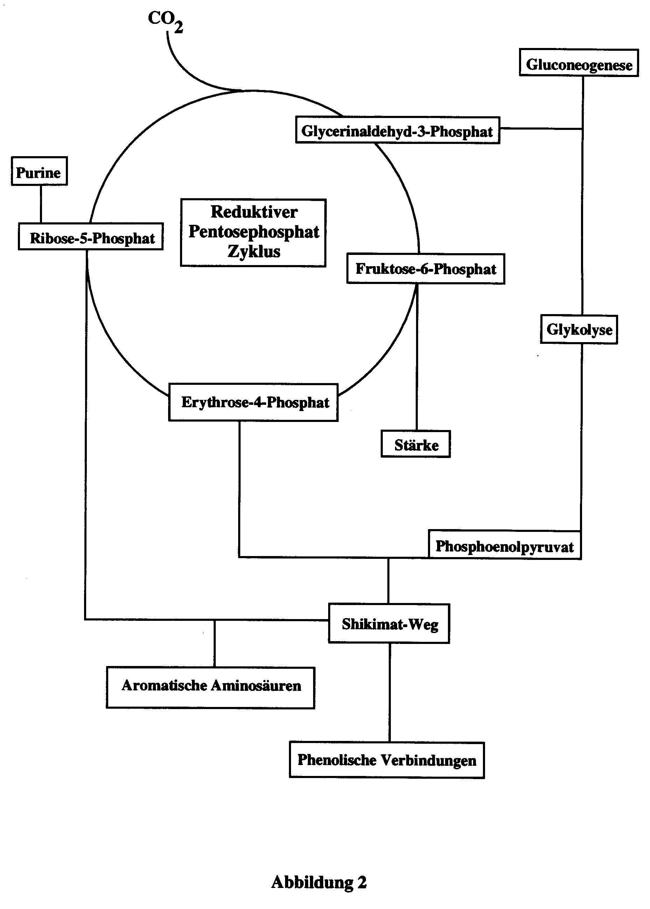

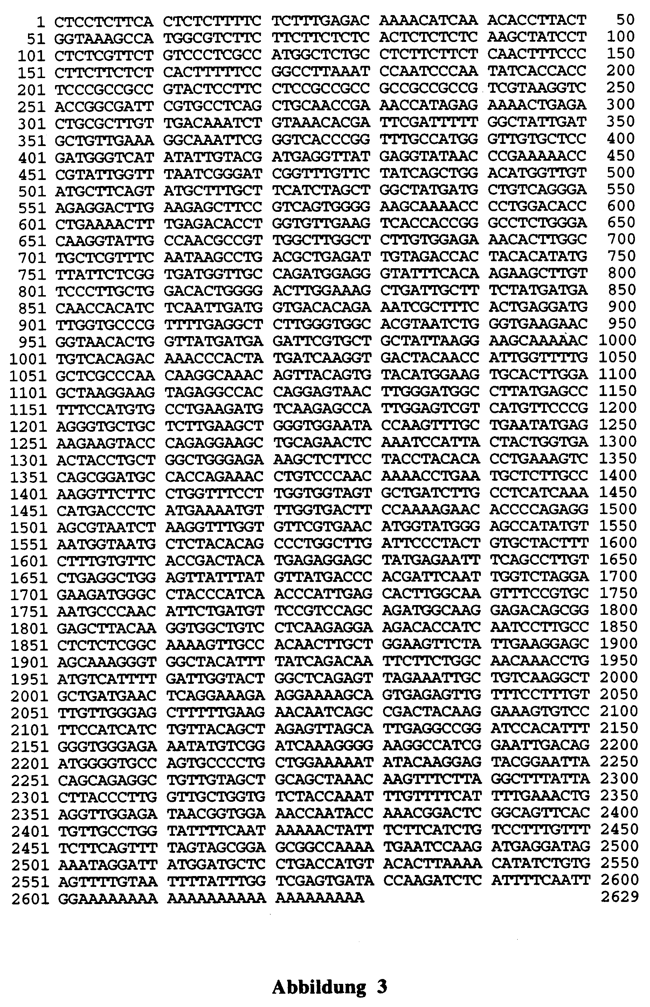

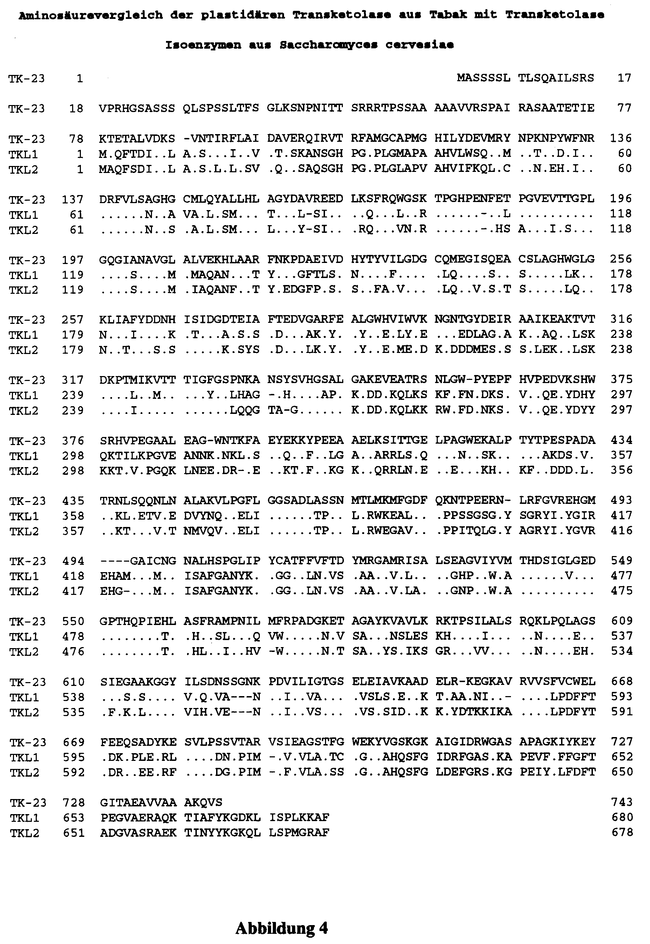

Homologievergleiche der abgeleiteten Aminosäuresequenz des Klons TK-23 (MacMolly Sequenzanalyse Programm von Macintosh) mit publizierten Transketolase-Sequenzen ergaben, daß im Bereich des vermutlich reifen Polypeptides (Aminosäure 78 bis 743) die höchsten Homologien zu Transketolasen aus Saccharomyces cervesiae bestehen (Abbildung 4). Die Sequenz des reifen Proteins (bestimmt durch Computervorhersage) ist zu 47,7 % bzw. 44,1 % identisch mit der Transketolase 1 bzw. 2 Sequenz aus Saccharomyces cervesiae. Geringere Sequenzhomologien wurden zu den übrigen Transketolasen gefunden. Keine Sequenzhomologie wurde für den Bereich des Transitpeptides ermittelt.

3. Expressionsanalyse der plastidären Transketolase

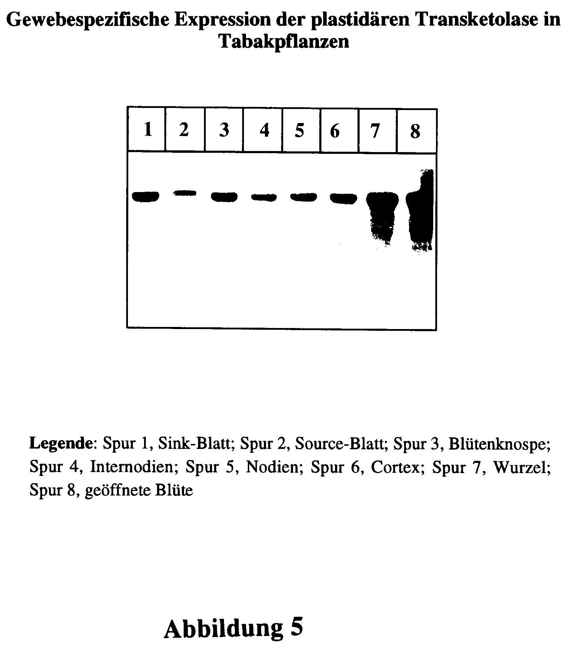

Expressionsanalysen einiger am Calvin Cyclus beteiligter Enzyme (RUBISCO, FBPase) haben ergeben, daß die Akkumulation der entsprechenden Transkripte an grünes Gewebe und Licht gebunden ist. Zur Überprüfung der gewebespezifischen Expression der Transketolase in Tabakpflanzen wurde Gesamt-RNA aus Sinkblättern, Sourceblättern, Blütenknospen, Stengeln (Internodien, Nodien und Mark), Wurzeln und geöffneten Blüten wachsender Tabakpflanzen isoliert. Nach Auftrennung in Agarosegelen und Bindung der RNA auf Nylonmembranen wurde die Anwesenheit Transketolase spezifischer Transkripte durch Hybridisierung mit der radioaktiven TK-23 cDNA nachgewiesen. Wie in Abbildung 5 dargestellt, sind Transketolase-spezifische Transkripte in allen getesteten Organen nachweisbar. Dieser Befund verdeutlicht, daß im Gegensatz zu anderen Enzymen des Calvin Cyclus, die Transketolase neben ihrer Funktion im Calvin Cyclus weitere Aufgaben im pflanzlichen Stoffwechsel erfüllt.

4. Antisenseinhibierung der Transketolase in transgenen Tabakpflanzen

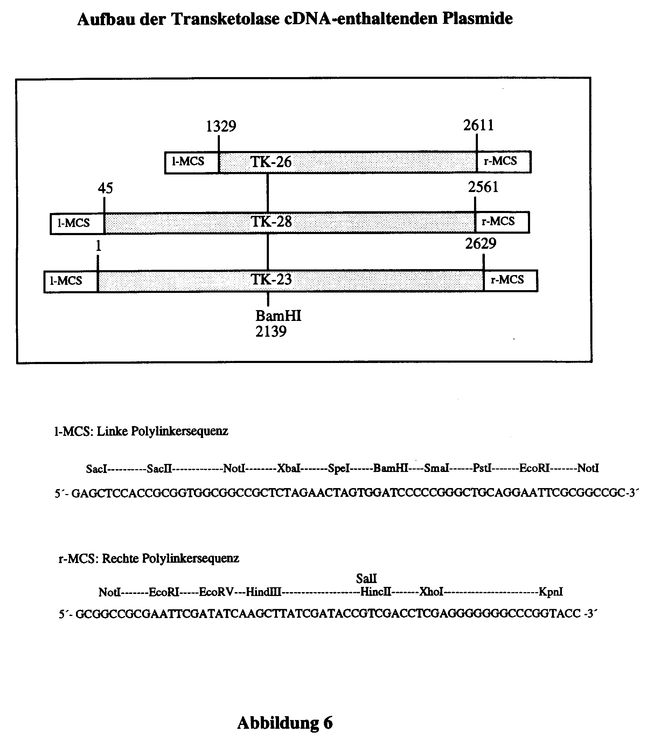

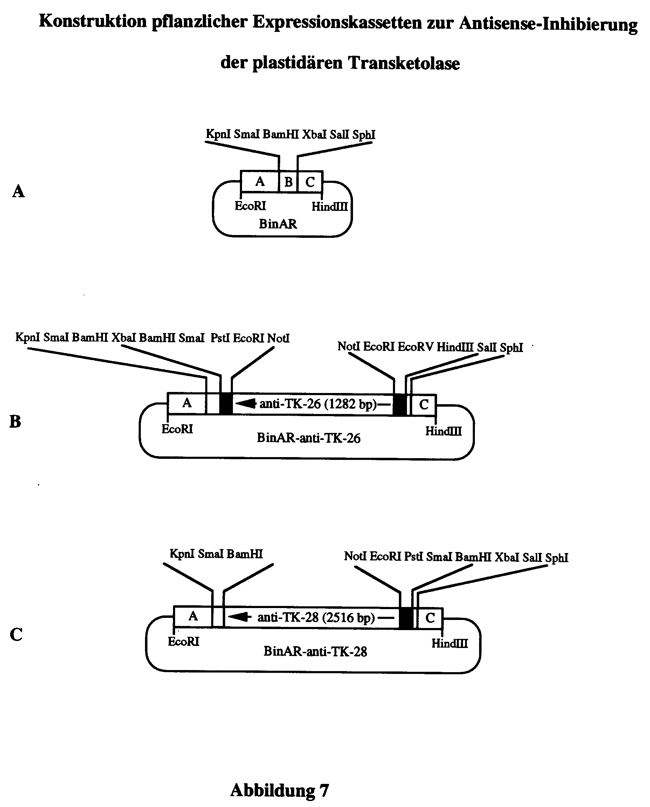

Um transgene Tabakpflanzen mit verminderter Transketolaseaktivität zu erzeugen, wurden die cDNA Klone TK-26 und TK-28 in Gegensinnrichtung mit einem eine konstitutive Expression bewirkenden Promotor sowie einem pflanzlichen Terminationssignal versehen. Die Plasmide BinAR-anti-TK-26 und BinAR-anti-TK-28 bestehend aus den drei Fragmenten A, der jeweiligen cDNA (s. Abb. 6, TK-26 und TK-28) und C wurden durch Insertion der entsprechenden cDNA Sequenzen in den Expressionsvektor pBinAR (Abb. 7A) erzeugt.

Das Fragment A beinhaltet den 35S CaMV Promoter. Es enthält ein Fragment, welches die Nukleotide 6909 bis 7437 des Cauliflower-Mosaik Virus (CaMV) umfaßt (Franck et al. (1980) Cell 21, 285). Es wurde als EcoRI-KpnI Fragment aus dem Plasmid pDH51 (Pietrzak et al. (1986) Nucleic. Acid Res. 14, 5857) isoliert. Die TK-26 cDNA wurde aus dem pBluescript SK- (Abb. 6) als XbaI-SalI Fragment und die TK-28 cDNA als BamHI Fragment in Gegensinnrichtung in den pBinAR Vektor kloniert (Abb. 7B und C). Das Fragment C enthält das Polyadenylierungssignal des Gens 3 der T-DNA des Ti-Plasmids pTiACH5 (Gielen et al. (1984); EMBO J. 3, 835), Nukleotide 11749-11939, welches als PvuII-HindIII Fragment aus dem Plasmid pAGV 40 (Herrera-Estrella et al. (1983); Nature 303,209) isoliert worden ist und nach Addition von SphI-Linkern an die PvuII-Schnittstelle zwischen die SpHI-HindIII Schnittstelle des Vektors kloniert worden war.

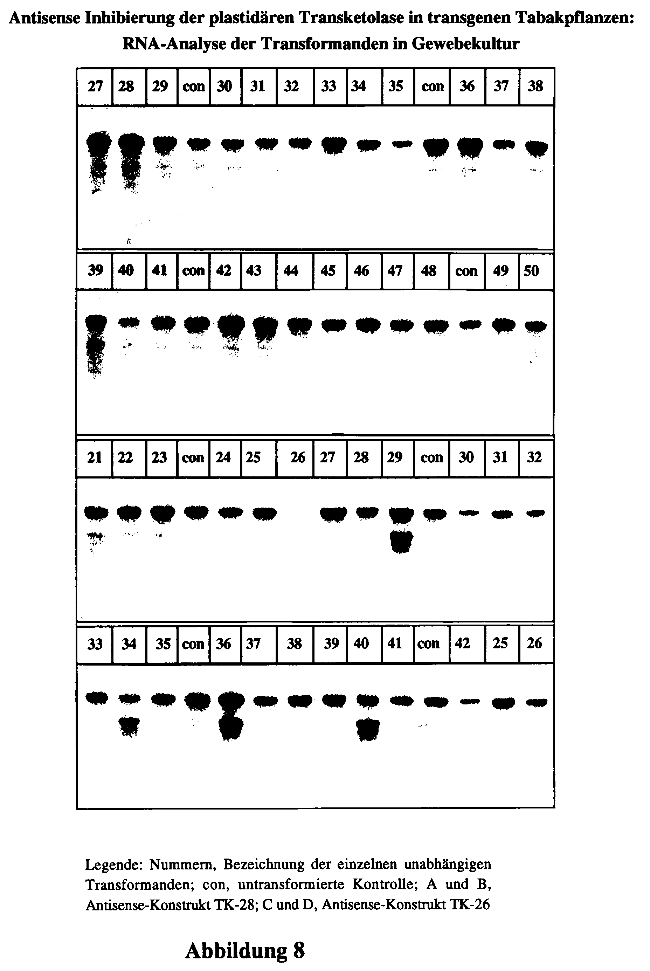

Die erhaltenen Plasmide wurden mit Hilfe des Agrobacteriumsystem in Tabak transformiert. Transformierte Tabakpflanzen wurden auf Antibiotika haltigem Medium angezogen und die erfolgreiche Inhibierung der Transketolase wurde durch Bestimmung der Transkriptmenge mittels Northern Experimenten ermittelt. Für jede Transformation (TK-26 und TK-28) wurden 100 unabhängige Transformanden untersucht. In Abbildung 8 ist das Ergebnis eines Northern-Experimentes dargestellt. In den meisten regenerierten Pflanzen konnte keine Reduktion der Transketolase mRNA nachgewiesen werde. Einige der Pflanzen zeigten allerdings eine starke Verminderung der Transketolase-spezifischen Transkripte (z.B. anti-TK-26 No. 26; Abb. 8). Die Reduktion der Transkriptmenge führte zu einer Unterdrückung des Pflanzenwachstums. Transfer der Pflanzen ins Gewächshaus führte zu einem Absterben der inhibierten Pflanzen.

5. Herstellung des Plasmides TK23-AC-pQE-9

Zur Etablierung eines molekularen Testsystems wurde die pflanzliche Transketolase in mikrobiellen Systemen überexprimiert.

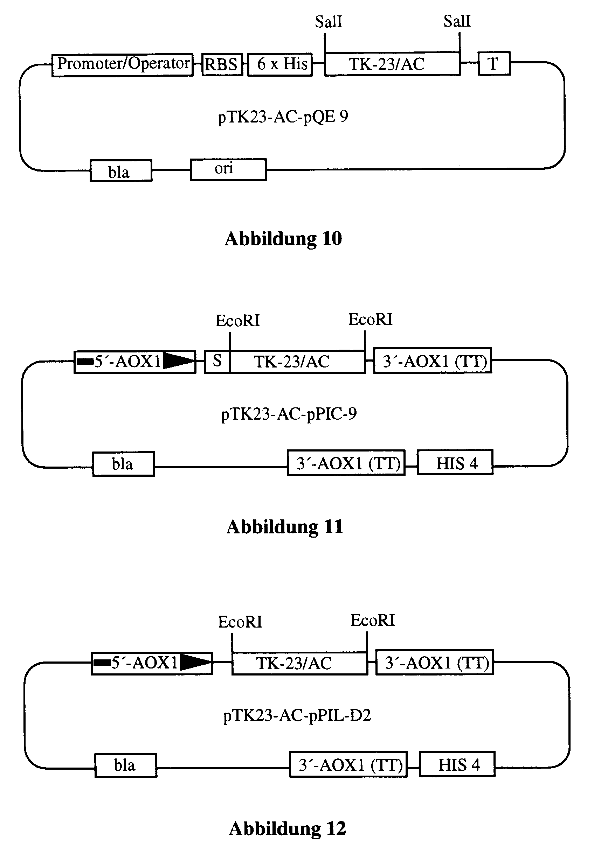

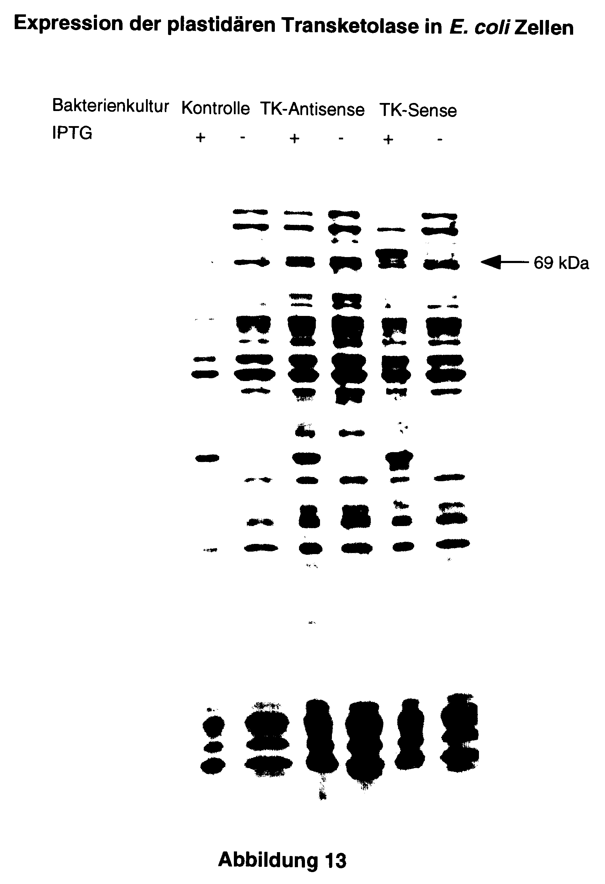

Zur Expression der Transketolase in E. coli wurde die TK-23 Sequenz, die für das reife Polypeptid kodiert, unter Verwendung der Primer A und C (s. Abb. 9) amplifiziert und in den Vektor pGEM-T kloniert (Gentechnische Verfahren, Absatz 8). Das TK23-AC PCR-Amplifikationsprodukt wurde anschließend als SalI Fragment in die SalI-Schnittstelle des Vektors PQE-9 (DIAGEN GmbH, QLAGEN Inc.) kloniert (Abb. 10).

6. Herstellung des Plasmides TK23-AC-pPIC-9 und TK23-BC-pHIL-D2

Da eukaryontische Enzyme häufig nur unzureichend in bakteriellen Systemen exprimiert werden können, wurden zwei weitere Plasmidkonstruktionen durchgeführt, die eine Expression in Pichia pastoris (Stamm GS115; Firma Invitrogen Corporation San Diego, CA 92121, USA) ermöglichen.

Zur Sekretion des Transketolase Proteins wurde das Plasmid TK23-AC-pPIC-9 konstruiert. Zur Fusion des Transketolase Proteins mit einem Hefe Signalpeptid wurde ein Teil der TK-23 Sequenz, der für das reife Polypeptid kodiert, unter Verwendung der Primer A und C (s. Abb. 9) amplifiziert und in den Vektor pGEM-T kloniert (Gentechnische Verfahren, Absatz 8). Das TK23-AC PCR-Amplifikationsprodukt wurde anschließend als EcoRI-Fragment in die EcoRI-Schnittstelle des Vektors pPIC-9 des Pichia Expressions Kit (Invitrogen) kloniert (Abb. 11). Um eine intrazelluläre Akkumulation des Transketolase Enzyms zu gewährleisten wurde das Plasmid TK23-BC-pHIL-D2 hergestellt. Zur besseren Aufreinigung des Enzyms wurde ein 5'-PCR Primer (s. Abb. 9) zur Amplifikation der Transketolase verwendet, der ein Startkodon für die Translation enthält und für sechs Histidinreste kodiert. Nach PCR-Amplifikation der in Abbildung 9 angegebenen TK-23 Sequenz wurde das TK-23-BC-Produkt in den Vektor pGEM-T kloniert. Das TK23-BC PCR-Amplifikationsprodukt wurde anschließend als EcoRI-Fragment in die EcoRI-Schnittstelle des Vektors pHIL-D2 des Pichia Expressions Kit (Invitrogen) kloniert (Abb. 12).

7. Expression der pflanzlichen Transketolase in E. coli

Zur Überexpression der Transketolase in E. coli wurden 2 ml LB-Medium mit XL-1 E-coli Zellen beimpft, die das Plasmid TK23-AC-pQU-9 enthielten. Die Kulturen wurden über Nacht bei 28°C in Anwesenheit von Antibiotika und unter Schütteln angezogenen. Anschließend wurden die Übernachtkulturen in 20 ml Wachstumsmedium (LB-Medium komplettiert mit: 10 µg/ml Tetracyclin, 200 µg/ml Ampicillin, 1 mM Vitamin B1, 1mM $MgSO_4$) überführt. Das Wachstum erfolgte bei 28°C unter Schütteln. Nach 3 Stunden wurde die Expression der Transketolase durch Zugabe von 2 mM IPTG induziert. Der Nachweis des produzierten Proteins wurde durch Auftrennung der Proteine in einem SDS-PAAG (Laemmli (1970) Nature 227, 680-685) mit anschließender Coomassie-Färbung der Proteine durchgeführt. Als Kontrollen dienten Kulturen, die entweder nicht mit IPTG induziert wurden, oder Kulturen, die die Transketolase in Gegensinnorientierung enthielten. Das Ergebnis eines Induktions-Experimentes ist in Abbildung 13 dargestellt. Ein Protein der entsprechenden Größe akkumulierte in Bakterienkulturen, die mit IPTG induziert wurden und das Plasmid TK23-AC-pQE-9 enthielten. Die Akkumulation beginnt eine Stunde nach Induktion. In den Kontrollen (ohne IPTG bzw. Transketolase in Gegensinnorientierung) ist kein vergleichbares Protein identifizierbar.

Abbildungen

1. Reduktiver Pentosephosphatzyklus
2. Verknüpfung des Pentosephosphatzyklus mit anderen Stoffwechselwegen
3. Nukleotidsequenz der plastidären Transketolase aus Tabak
4. Aminosäurevergleich der plastidären Transketolase mit Transketolase 1 und 2 aus Hefe
5. Nachweis der Transketolase mRNA in unterschiedlichen Tabakgeweben
6. Schematische Darstellung der Transketolase cDNA-Klone
7. Schematische Darstellung der Plasmide BinAR-TK-26-anti und BinAR-TK-28-anu
8. Northernanalyse transgener Tabakpflanzen
9. Strategie und Oligonukleotide zur PCR-Amplifikation der plastidären Transketolase
10. Schematische Darstellung des Plasmides TK23-AC-pQE-9
11. Schematische Darstellung des Plasmides TK23-AC-pPIC-9
12. Schematische Darstellung des Plasmides TK23-BC-pHIL-D2
13. Überexpression der pflanzlichen Transketolase in E. coli

**Patentansprüche**

1. Protein mit Transketolase Aktivität, enthaltend eine Aminosäuresequenz, die eine Teilsequenz von mindestens 100 Aminosäuren aus SEQ ID NO 2 darstellt.

2. Protein nach Anspruch 1, dadurch gekennzeichnet, daß es als Aminosäuresequenz die Teilsequenz 78-743 aus SEQ ID NO 2 enthält.

3. Protein nach Anspruch 2, dadurch gekennzeichnet, daß es als Aminosäuresequenz die in SEQ ID NO 2 dargestellte Sequenz enthält.

4. Nukleinsäure, codierend für ein Protein gemäß einem der Ansprüche 1-3.

5. Nukleinsäure nach Anspruch 4, dadurch gekennzeichnet, daß sie aus der in SEQ ID NO 1 dargestellten Sequenz besteht.

6. Vektoren, enthaltend eine Nukleinsäure gemäß Anspruch 4 oder 5 zusammen mit funktionellen Regulationssignalen.

7. Verwendung eines Proteins gemäß Anspruch 1-3 zur Identifizierung von herbiziden Wirkstoffen.

8. Verwendung nach Anspruch 7, dadurch gekennzeichnet,daß die Identifizierung mittels eines in vitro Testsystems erfolgt.

9. Verwendung nach Anspruch 8, dadurch gekennzeichnet, daß als Testsystem ein Enzymhemmtest eingesetzt wird.

10. Testsystem zur Identifizierung von Transketolase-Inhibitoren, dadurch gekennzeichnet, daß man die potentiellen Inhibitoren mit einem Protein gemäß Anspruch 1-3 inkubiert und anschließend die Transketolase Aktivität bestimmt.

11. Herbizide Wirkstoffe, identifizierbar mittels eines Testsystems gemäß Anspruch 10.

12. Verfahren zur Herstellung von Herbiziden, die eine pflanzliche Transketolase inhibieren, dadurch gekennzeichnet, daß man bekanntechemische Verbindungen in einem Testverfahren gemäß Anspruch 10 überprüft und solche mit inhibierender Wirkung mit üblichen Träger- und Hilfsstoffen als Herbizid formuliert.

**Abbildung 1**

$CO_2$

Gluconeogenese

Glycerinaldehyd-3-Phosphat

Purine

Ribose-5-Phosphat

Reduktiver
Pentosephosphat
Zyklus

Fruktose-6-Phosphat

Glykolyse

Erythrose-4-Phosphat

Stärke

Phosphoenolpyruvat

Shikimat-Weg

Aromatische Aminosäuren

Phenolische Verbindungen

Abbildung 2

17

```
   1 CTCCTCTTCA CTCTCTTTTC TCTTTGAGAC AAAACATCAA ACACCTTACT   50
  51 GGTAAAGCCA TGGCGTCTTC TTCTTCTCTC ACTCTCTCTC AAGCTATCCT  100
 101 CTCTCGTTCT GTCCCTCGCC ATGGCTCTGC CTCTTCTTCT CAACTTTCCC  150
 151 CTTCTTCTCT CACTTTTTCC GGCCTTAAAT CCAATCCCAA TATCACCACC  200
 201 TCCCGCCGCC GTACTCCTTC CTCCGCCGCC GCCGCCGCCG TCGTAAGGTC  250
 251 ACCGGCGATT CGTGCCTCAG CTGCAACCGA AACCATAGAG AAAACTGAGA  300
 301 CTGCGCTTGT TGACAAATCT GTAAACACGA TTCGATTTTT GGCTATTGAT  350
 351 GCTGTTGAAA GGCAAATTCG GGTCACCCGG TTTGCCATGG GTTGTGCTCC  400
 401 GATGGGTCAT ATATTGTACG ATGAGGTTAT GAGGTATAAC CCGAAAAACC  450
 451 CGTATTGGTT TAATCGGGAT CGGTTTGTTC TATCAGCTGG ACATGGTTGT  500
 501 ATGCTTCAGT ATGCTTTGCT TCATCTAGCT GGCTATGATG CTGTCAGGGA  550
 551 AGAGGACTTG AAGAGCTTCC GTCAGTGGGG AAGCAAAACC CCTGGACACC  600
 601 CTGAAAACTT TGAGACACCT GGTGTTGAAG TCACCACCGG GCCTCTGGGA  650
 651 CAAGGTATTG CCAACGCCGT TGGCTTGGCT CTTGTGGAGA AACACTTGGC  700
 701 TGCTCGTTTC AATAAGCCTG ACGCTGAGAT TGTAGACCAC TACACATATG  750
 751 TTATTCTCGG TGATGGTTGC CAGATGGAGG GTATTTCACA AGAAGCTTGT  800
 801 TCCCTTGCTG GACACTGGGG ACTTGGAAAG CTGATTGCTT TCTATGATGA  850
 851 CAACCACATC TCAATTGATG GTGACACAGA AATCGCTTTC ACTGAGGATG  900
 901 TTGGTGCCCG TTTTGAGGCT CTTGGGTGGC ACGTAATCTG GGTGAAGAAC  950
 951 GGTAACACTG GTTATGATGA GATTCGTGCT GCTATTAAGG AAGCAAAAAC 1000
1001 TGTCACAGAC AAACCCACTA TGATCAAGGT GACTACAACC ATTGGTTTTG 1050
1051 GCTCGCCCAA CAAGGCAAAC AGTTACAGTG TACATGGAAG TGCACTTGGA 1100
1101 GCTAAGGAAG TAGAGGCCAC CAGGAGTAAC TTGGGATGGC CTTATGAGCC 1150
1151 TTTCCATGTG CCTGAAGATG TCAAGAGCCA TTGGAGTCGT CATGTTCCCG 1200
1201 AGGGTGCTGC TCTTGAAGCT GGGTGGAATA CCAAGTTTGC TGAATATGAG 1250
1251 AAGAAGTACC CAGAGGAAGC TGCAGAACTC AAATCCATTA CTACTGGTGA 1300
1301 ACTACCTGCT GGCTGGGAGA AAGCTCTTCC TACCTACACA CCTGAAAGTC 1350
1351 CAGCGGATGC CACCAGAAAC CTGTCCCAAC AAAACCTGAA TGCTCTTGCC 1400
1401 AAGGTTCTTC CTGGTTTCCT TGGTGGTAGT GCTGATCTTG CCTCATCAAA 1450
1451 CATGACCCTC ATGAAAATGT TTGGTGACTT CCAAAAGAAC ACCCCAGAGG 1500
1501 AGCGTAATCT AAGGTTTGGT GTTCGTGAAC ATGGTATGGG AGCCATATGT 1550
1551 AATGGTAATG CTCTACACAG CCCTGGCTTG ATTCCCTACT GTGCTACTTT 1600
1601 CTTTGTGTTC ACCGACTACA TGAGAGGAGC TATGAGAATT TCAGCCTTGT 1650
1651 CTGAGGCTGG AGTTATTTAT GTTATGACCC ACGATTCAAT TGGTCTAGGA 1700
1701 GAAGATGGGC CTACCCATCA ACCCATTGAG CACTTGGCAA GTTTCCGTGC 1750
1751 AATGCCCAAC ATTCTGATGT TCCGTCCAGC AGATGGCAAG GAGACAGCGG 1800
1801 GAGCTTACAA GGTGGCTGTC CTCAAGAGGA AGACACCATC AATCCTTGCC 1850
1851 CTCTCTCGGC AAAAGTTGCC ACAACTTGCT GGAAGTTCTA TTGAAGGAGC 1900
1901 AGCAAAGGGT GGCTACATTT TATCAGACAA TTCTTCTGGC AACAAACCTG 1950
1951 ATGTCATTTT GATTGGTACT GGCTCAGAGT TAGAAATTGC TGTCAAGGCT 2000
2001 GCTGATGAAC TCAGGAAAGA AGGAAAAGCA GTGAGAGTTG TTTCCTTTGT 2050
2051 TTGTTGGGAG CTTTTTGAAG AACAATCAGC CGACTACAAG GAAAGTGTCC 2100
2101 TTCCATCATC TGTTACAGCT AGAGTTAGCA TTGAGGCCGG ATCCACATTT 2150
2151 GGGTGGAGA AATATGTCGG ATCAAAGGGG AAGGCCATCG GAATTGACAG 2200
2201 ATGGGGTGCC AGTGCCCCTG CTGGAAAAAT ATACAAGGAG TACGGAATTA 2250
2251 CAGCAGAGGC TGTTGTAGCT GCAGCTAAAC AAGTTTCTTA GGCTTTATTA 2300
2301 CTTACCCTTG GTTGCTGGTG TCTACCAAAT TTGTTTTCAT TTTGAAACTG 2350
2351 AGGTTGGAGA TAACGGTGGA AACCAATACC AAACGGACTC GGCAGTTCAC 2400
2401 TGTTGCCTGG TATTTTCAAT AAAAACTATT TCTTCATCTG TCCTTTGTTT 2450
2451 TCTTCAGTTT TAGTAGCGGA GCGGCCAAAA TGAATCCAAG ATGAGGATAG 2500
2501 AAATAGGATT ATGGATGCTC CTGACCATGT ACACTTAAAA CATATCTGTG 2550
2551 AGTTTTGTAA TTTTATTTGG TCGAGTGATA CCAAGATCTC ATTTTCAATT 2600
2601 GGAAAAAAAA AAAAAAAAAA AAAAAAAA                          2629
```

**Abbildung 3**

Aminosäurevergleich der plastidären Transketolase aus Tabak mit Transketolase

Isoenzymen aus Saccharomyces cervesiae

```
TK-23    1                                                       MASSSSL TLSQAILSRS    17

TK-23   18  VPRHGSASSS QLSPSSLTFS GLKSNPNITT SRRRTPSSAA AAAVVRSPAI RASAATETIE    77

TK-23   78  KTETALVDKS -VNTIRFLAI DAVERQIRVT RFAMGCAPMG HILYDEVMRY NPKNPYWFNR   136
TKL1     1  M.QFTDI..L A.S...I..V .T.SKANSGH PG.PLGMAPA AHVLWSQ..M ..T..D.I..    60
TKL2     1  MAQFSDI..L A.S.L.L.SV .Q..SAQSGH PG.PLGLAPV AHVIFKQL.C ..N.EH.I..    60

TK-23  137  DRFVLSAGHG CMLQYALLHL AGYDAVREED LKSFRQWGSK TPGHPENFET PGVEVTTGPL   196
TKL1    61  ......N..A VA.L.SM... T...L-SI.. ..Q...L..R .......-..L ..........   118
TKL2    61  ......N..S .A.L.SM... L...Y-SI.. .RQ...VN.R .......-.HS A...I.S...   118

TK-23  197  GQGIANAVGL ALVEKHLAAR FNKPDAEIVD HYTYVILGDG CQMEGISQEA CSLAGHWGLG   256
TKL1   119  ....S....M .MAQAN...T Y...GFTLS. N....F.... .LQ....S.. S.....LK..   178
TKL2   119  ....S....M .IAQANF..T Y.EDGFP.S. S..FA.V... .LQ..V.S.T S.....LQ..   178

TK-23  257  KLIAFYDDNH ISIDGDTEIA FTEDVGARFE ALGWHVIWVK NGNTGYDEIR AAIKEAKTVT   316
TKL1   179  N...I....K .T...A.S.S .D...AK.Y. .Y..E.LY.E ...EDLAG.A K..AQ..LSK   238
TKL2   179  N..T...S.S .....K.SYS .D...LK.Y. .Y..E.ME.D K.DDDMES.S S.LEK..LSK   238

TK-23  317  DKPTMIKVTT TIGFGSPNKA NSYSVHGSAL GAKEVEATRS NLGW-PYEPF HVPEDVKSHW   375
TKL1   239  ....L..M.. ...Y..LHAG -.H....AP. K.DD.KQLKS KF.FN.DKS. V..QE.YDHY   297
TKL2   239  ....I..... ......LQQG TA-G...... K.DD.KQLKK RW.FD.NKS. V..QE.YDYY   297

TK-23  376  SRHVPEGAAL EAG-WNTKFA EYEKKYPEEA AELKSITTGE LPAGWEKALP TYTPESPADA   434
TKL1   298  QKTILKPGVE ANNK.NKL.S ..Q..F..LG A..ARRLS.Q ...N..SK.. ...AKDS.V.   357
TKL2   298  KKT.V.PGQK LNEE.DR-.E ..KT.F..KG K..QRRLN.E ..E...KH.. KF..DDD.L.   356

TK-23  435  TRNLSQQNLN ALAKVLPGFL GGSADLASSN MTLMKMFGDF QKNTPEERN- LRFGVREHGM   493
TKL1   358  ..KL.ETV.E DVYNQ..ELI ......TP.. L.RWKEAL.. .PPSSGSG.Y SGRYI.YGIR   417
TKL2   357  ..KT...V.T NMVQV..ELI ......TP.. L.RWEGAV.. .PPITQLG.Y AGRYI.YGVR   416

TK-23  494  ----GAICNG NALHSPGLIP YCATFFVFTD YMRGAMRISA LSEAGVIYVM THDSIGLGED   549
TKL1   418  EHAM...M.. ISAFGANYK. .GG..LN.VS .AA..V.L.. ..GHP..W.A ......V...   477
TKL2   417  EHG-...M.. ISAFGANYK. .GG..LN.VS .AA..V.LA. ..GNP..W.A ..........   475

TK-23  550  GPTHQPIEHL ASFRAMPNIL MFRPADGKET AGAYKVAVLK RKTPSILALS RQKLPQLAGS   609
TKL1   478  ........T. .H..SL...Q VW.....N.V SA...NSLES KH....I... ..N....E..   537
TKL2   476  ........T. .HL..I..HV -W.....N.T SA..YS.IKS GR...VV... ..N....EH.   534

TK-23  610  SIEGAAKGGY ILSDNSSGNK PDVILIGTGS ELEIAVKAAD ELR-KEGKAV RVVSFVCWEL   668
TKL1   538  ...S.S.... V.Q.VA---N ..I..VA... .VSLS.E..K T.AA.NI..- ....LPDFFT   593
TKL2   535  .F.K.L.... VIH.VE---N ..I..VS... .VS.SID..K K.YDTKKIKA ....LPDFYT   591

TK-23  669  FEEQSADYKE SVLPSSVTAR VSIEAGSTFG WEKYVGSKGK AIGIDRWGAS APAGKIYKEY   727
TKL1   595  .DK.PLE.RL ....DN.PIM -.V.VLA.TC .G..AHQSFG IDRFGAS.KA PEVF.FFGFT   652
TKL2   592  .DR..EE.RF ....DG.PIM -.F.VLA.SS .G..AHQSFG LDEFGRS.KG PEIY.LFDFT   650

TK-23  728  GITAEAVVAA AKQVS                                                   743
TKL1   653  PEGVAERAQK TIAFYKGDKL ISPLKKAF                                     680
TKL2   651  ADGVASRAEK TINYYKGKQL LSPMGRAF                                     678
```

**Abbildung 4**

**Gewebespezifische Expression der plastidären Transketolase in Tabakpflanzen**

**Legende**: Spur 1, Sink-Blatt; Spur 2, Source-Blatt; Spur 3, Blütenknospe; Spur 4, Internodien; Spur 5, Nodien; Spur 6, Cortex; Spur 7, Wurzel; Spur 8, geöffnete Blüte

# Abbildung 5

**Aufbau der Transketolase cDNA-enthaltenden Plasmide**

l-MCS: Linke Polylinkersequenz

SacI---------SacII------------NotI--------XbaI-------SpeI------BamHI----SmaI------PstI-------EcoRI-------NotI

5'- GAGCTCCACCGCGGTGGCGGCCGCTCTAGAACTAGTGGATCCCCCGGGCTGCAGGAATTCGCGGCCGC-3'

r-MCS: Rechte Polylinkersequenz

                                                 SalI

NotI-------EcoRI-----EcoRV---HindIII------------------HincII-------XhoI----------------------KpnI

5'- GCGGCCGCGAATTCGATATCAAGCTTATCGATACCGTCGACCTCGAGGGGGGGGCCCGGTACC -3'

**Abbildung 6**

**Konstruktion pflanzlicher Expressionskassetten zur Antisense-Inhibierung**

**der plastidären Transketolase**

**Abbildung 7**

**Antisense Inhibierung der plastidären Transketolase in transgenen Tabakpflanzen: RNA-Analyse der Transformanden in Gewebekultur**

Legende: Nummern, Bezeichnung der einzelnen unabhängigen Transformanden; con, untransformierte Kontrolle; A und B, Antisense-Konstrukt TK-28; C und D, Antisense-Konstrukt TK-26

**Abbildung 8**

## PCR-Amplifikation der plastidären Transketolase

EP 0 723 017 A2

PCR-Primer:

A: 5´- AA GTC GAC GAA TTC AAA ATC GAG ACT GCG CTT GTT GAC -3´
             SalI       EcoRI   TK reifes Protein
38mer

B: 5´- AA GAA TTC ATG CAT CAT CAT CAT CAT CAT AAA ATC GAG ACT GCG CTT GTT GAC -3´
           EcoRI   Met           6 x His          TK reifes Protein
53mer

C: 5´- TT GTC GAC GAA TTC CTA AGA AAC TTG TTT AGC TGC AGC -3´
             SalI       EcoRI  Stop
38mer

**Abbildung 9**

SalI                    SalI

| Promoter/Operator | RBS | 6 x His | TK-23/AC | T |

pTK23-AC-pQE 9

| bla | | ori |

**Abbildung 10**

EcoRI                    EcoRI

| 5´-AOX1 | S | TK-23/AC | 3´-AOX1 (TT) |

pTK23-AC-pPIC-9

| bla | | 3´-AOX1 (TT) | HIS 4 |

**Abbildung 11**

EcoRI              EcoRI

| 5´-AOX1 | TK-23/AC | 3´-AOX1 (TT) |

pTK23-AC-pPIL-D2

| bla | | 3´-AOX1 (TT) | HIS 4 |

**Abbildung 12**

# Expression der plastidären Transketolase in *E. coli* Zellen

Bakterienkultur  Kontrolle  TK-Antisense  TK-Sense

IPTG            +    -     +    -     +    -

← 69 kDa

**Abbildung 13**

SEQUENZPROTOKOLL

(1)  ALLGEMEINE INFORMATION:

   (i)     ANMELDER:

   (A)     NAME: BASF Aktiengesellschaft

   (B)     STRASSE: Carl-Bosch-Strasse 38

   (C)     ORT: Ludwigshafen

   (E)     LAND: Bundesrepublik Deutschland

   (F)     POSTLEITZAHL: D-67056

   (G)     TELEPHON: 0621/6048526

   (H)     TELEFAX: 0621/6043123

   (I)     TELEX: 1762175170

   (ii)    ANMELDETITEL: Transketolase aus Pflanzen
   (iii)   ANZAHL DER SEQUENZEN: 2
   (iv)    COMPUTER-LESBARE FORM:
           (A) DATENTRÄGER: Floppy disk
           (B) COMPUTER: IBM PC compatible
           (C) BETR1EBSSYSTEM: PC-DOS/MS-DOS
           (D) SOFTWARE: PatentIn Release #1.0, Version #1.25 (EPA)

(2) INFORMATION ZU SEQ ID NO: 1:
   (i)     SEQUENZ CHARAKTERISTIKA:
                   (A) LÄNGE: 2629 Basenpaare
                   (B) ART: Nukleinsäure
                   (C) STRANGFORM: Einzel
                   (D) TOPOLOGIE: linear
   (ii)    ART DES MOLEKüLS: cDNS
   (iii)   HYPOTHETISCH: NEIN
   (vi)    URSPRÜNGLICHE HERKUNFT:
           (A) ORGANISMUS: Nicotiana
   (ix)    MERKMALE:
           (A) NAME/SCHLÜSSEL: CDS
           (B) LAGE: 60..2289
   (xi)    SEQUENZBESCHREIBUNG: SEQ ID NO: 1:

8

```
CTCCTCTTCA CTCTCTTTTC TCTTTGAGAC AAAACATCAA ACACCTTACT GGTAAAGCC        59

ATG GCG TCT TCT TCT TCT CTC ACT CTC TCT CAA GCT ATC CTC TCT CGT        107
Met Ala Ser Ser Ser Ser Leu Thr Leu Ser Gln Ala Ile Leu Ser Arg
  1               5                  10                  15

TCT GTC CCT CGC CAT GGC TCT GCC TCT TCT TCT CAA CTT TCC CCT TCT        155
Ser Val Pro Arg His Gly Ser Ala Ser Ser Ser Gln Leu Ser Pro Ser
              20                  25                  30

TCT CTC ACT TTT TCC GGC CTT AAA TCC AAT CCC AAT ATC ACC ACC TCC        203
Ser Leu Thr Phe Ser Gly Leu Lys Ser Asn Pro Asn Ile Thr Thr Ser
          35                  40                  45

CGC CGC CGT ACT CCT TCC TCC GCC GCC GCC GCC GCC GTC GTA AGG TCA        251
Arg Arg Arg Thr Pro Ser Ser Ala Ala Ala Ala Ala Val Val Arg Ser
      50                  55                  60

CCG GCG ATT CGT GCC TCA GCT GCA ACC GAA ACC ATA GAG AAA ACT GAG        299
Pro Ala Ile Arg Ala Ser Ala Ala Thr Glu Thr Ile Glu Lys Thr Glu
  65                  70                  75                  80

ACT GCG CTT GTT GAC AAA TCT GTA AAC ACG ATT CGA TTT TTG GCT ATT        347
Thr Ala Leu Val Asp Lys Ser Val Asn Thr Ile Arg Phe Leu Ala Ile
                  85                  90                  95

GAT GCT GTT GAA AGG CAA ATT CGG GTC ACC CGG TTT GCC ATG GGT TGT        395
Asp Ala Val Glu Arg Gln Ile Arg Val Thr Arg Phe Ala Met Gly Cys
              100                 105                 110

GCT CCG ATG GGT CAT ATA TTG TAC GAT GAG GTT ATG AGG TAT AAC CCG        443
Ala Pro Met Gly His Ile Leu Tyr Asp Glu Val Met Arg Tyr Asn Pro
          115                 120                 125

AAA AAC CCG TAT TGG TTT AAT CGG GAT CGG TTT GTT CTA TCA GCT GGA        491
Lys Asn Pro Tyr Trp Phe Asn Arg Asp Arg Phe Val Leu Ser Ala Gly
          130                 135                 140

CAT GGT TGT ATG CTT CAG TAT GCT TTG CTT CAT CTA GCT GGC TAT GAT        539
His Gly Cys Met Leu Gln Tyr Ala Leu Leu His Leu Ala Gly Tyr Asp
145                 150                 155                 160

GCT GTC AGG GAA GAG GAC TTG AAG AGC TTC CGT CAG TGG GGA ACC AAA        587
Ala Val Arg Glu Glu Asp Leu Lys Ser Phe Arg Gln Trp Gly Thr Lys
              165                 170                 175

ACC CCT GGA CAC CCT GAA AAC TTT GAG ACA CCT GGT GTT GAA GTC ACC        635
Thr Pro Gly His Pro Glu Asn Phe Glu Thr Pro Gly Val Glu Val Thr
              180                 185                 190

ACC GGG CCT CTG GGA CAA GGT ATT GCC AAC GCC GTT GGC TTG GCT CTT        683
Thr Gly Pro Leu Gly Gln Gly Ile Ala Asn Ala Val Gly Leu Ala Leu
          195                 200                 205

GTG GAG AAA CAC TTG GCT GCT CGT TTC AAT AAG CCT GAC GCT GAG ATT        731
Val Glu Lys His Leu Ala Ala Arg Phe Asn Lys Pro Asp Ala Glu Ile
      210                 215                 220

GTA GAC CAC TAC ACA TAT GTT ATT CTC GGT GAT GGT TGC CAG ATG GAG        779
Val Asp His Tyr Thr Tyr Val Ile Leu Gly Asp Gly Cys Gln Met Glu
225                 230                 235                 240
```

9

```
GGT ATT TCA CAA GAA GCT TGT TCC CTT GCT GGA CAC TGG GGA CTT GGA        827
Gly Ile Ser Gln Glu Ala Cys Ser Leu Ala Gly His Trp Gly Leu Gly
                245                 250                 255
AAG CTG ATT GCT TTC TAT GAT GAC AAC CAC ATC TCA ATT GAT GGT GAC        875
Lys Leu Ile Ala Phe Tyr Asp Asp Asn His Ile Ser Ile Asp Gly Asp
                260                 265                 270
ACA GAA ATC GCT TTC ACT GAG GAT GTT GGT GCC CGT TTT GAG GCT CTT        923
Thr Glu Ile Ala Phe Thr Glu Asp Val Gly Ala Arg Phe Glu Ala Leu
                275                 280                 285
GGG TGG CAC GTA ATC TGG GTG AAG AAC GGT AAC ACT GGT TAT GAT GAG        971
Gly Trp His Val Ile Trp Val Lys Asn Gly Asn Thr Gly Tyr Asp Glu
                290                 295                 300
ATT CGT GCT GCT ATT AAG GAA GCA AAA ACT GTC ACA GAC AAA CCC ACT       1019
Ile Arg Ala Ala Ile Lys Glu Ala Lys Thr Val Thr Asp Lys Pro Thr
305                 310                 315                 320
ATG ATC AAG GTG ACT ACA ACC ATT GGT TTT GGC TCG CCC AAC AAG GCA       1067
Met Ile Lys Val Thr Thr Thr Ile Gly Phe Gly Ser Pro Asn Lys Ala
                325                 330                 335
AAC AGT TAC AGT GTA CAT GGA AGT GCA CTT GGA GCT AAG GAA GTA GAG       1115
Asn Ser Tyr Ser Val His Gly Ser Ala Leu Gly Ala Lys Glu Val Glu
                340                 345                 350
GCC ACC AGG AGT AAC TTG GGA TGG CCT TAT GAG CCT TTC CAT GTG CCT       1163
Ala Thr Arg Ser Asn Leu Gly Trp Pro Tyr Glu Pro Phe His Val Pro
                355                 360                 365
GAA GAT GTC AAG AGC CAT TGG AGT CGT CAT GTT CCC GAG GGT GCT GCT       1211
Glu Asp Val Lys Ser His Trp Ser Arg His Val Pro Glu Gly Ala Ala
                370                 375                 380
CTT GAA GCT GGG TGG AAT ACC AAG TTT GCT GAA TAT GAG AAG AAG TAC       1259
Leu Glu Ala Gly Trp Asn Thr Lys Phe Ala Glu Tyr Glu Lys Lys Tyr
385                 390                 395                 400
CCA GAG GAA GCT GCA GAA CTC AAA TCC ATT ACT ACT GGT GAA CTA CCT       1307
Pro Glu Glu Ala Ala Glu Leu Lys Ser Ile Thr Thr Gly Glu Leu Pro
                405                 410                 415
GCT GGC TGG GAG AAA GCT CTT CCT ACC TAC ACA CCT GAA AGT CCA GCG       1355
Ala Gly Trp Glu Lys Ala Leu Pro Thr Tyr Thr Pro Glu Ser Pro Ala
                420                 425                 430
GAT GCC ACC AGA AAC CTG TCC CAA CAA AAC CTG AAT GCT CTT GCC AAG       1403
Asp Ala Thr Arg Asn Leu Ser Gln Gln Asn Leu Asn Ala Leu Ala Lys
                435                 440                 445
GTT CTT CCT GGT TTC CTT GGT GGT AGT GCT GAT CTT GCC TCA TCA AAC       1451
Val Leu Pro Gly Phe Leu Gly Gly Ser Ala Asp Leu Ala Ser Ser Asn
                450                 455                 460
ATG ACC CTC ATG AAA ATG TTT GGT GAC TTC CAA AAG AAC ACC CCA GAG       1499
Met Thr Leu Met Lys Met Phe Gly Asp Phe Gln Lys Asn Thr Pro Glu
465                 470                 475                 480
```

```
GAG CGT AAT CTA AGG TTT GGT GTT CGT GAA CAT GGT ATG GGA GCC ATA          1547
Glu Arg Asn Leu Arg Phe Gly Val Arg Glu His Gly Met Gly Ala Ile
                485                 490                 495
TGT AAT GGT AAT GCT CTA CAC AGC CCT GGC TTG ATT CCC TAC TGT GCT          1595
Cys Asn Gly Asn Ala Leu His Ser Pro Gly Leu Ile Pro Tyr Cys Ala
                500                 505                 510
ACT TTC TTT GTG TTC ACC GAC TAC ATG AGA GGA GCT ATG AGA ATT TCA          1643
Thr Phe Phe Val Phe Thr Asp Tyr Met Arg Gly Ala Met Arg Ile Ser
                515                 520                 525
GCC TTG TCT GAG GCT GGA GTT ATT TAT GTT ATG ACC CAC GAT TCA ATT          1691
Ala Leu Ser Glu Ala Gly Val Ile Tyr Val Met Thr His Asp Ser Ile
                530                 535                 540
GGT CTA GGA GAA GAT GGG CCT ACC CAT CAA CCC ATT GAG CAC TTG CCA          1739
Gly Leu Gly Glu Asp Gly Pro Thr His Gln Pro Ile Glu His Leu Pro
545                 550                 555                 560
AGT TTC CGT GCA ATG CCC AAC ATT CTG ATG TTC CGT CCA GCA GAT GGC          1787
Ser Phe Arg Ala Met Pro Asn Ile Leu Met Phe Arg Pro Ala Asp Gly
                565                 570                 575
AAG GAG ACA GCG GGA GCT TAC AAG GTG GCT GTC CTC AAG AGG AAG ACA          1835
Lys Glu Thr Ala Gly Ala Tyr Lys Val Ala Val Leu Lys Arg Lys Thr
                580                 585                 590
CCA TCA ATC CTT GCC CTC TCT CGG CAA AAG TTG CCA CAA CTT GCT GGA          1883
Pro Ser Ile Leu Ala Leu Ser Arg Gln Lys Leu Pro Gln Leu Ala Gly
                595                 600                 605
AGT TCT ATT GAA GGA GCA GCA AAG CGT GGC TAC ATT TTA TCA GAC AAT          1931
Ser Ser Ile Glu Gly Ala Ala Lys Arg Gly Tyr Ile Leu Ser Asp Asn
                610                 615                 620
TCT TCT GGC AAC AAA CCT GAT GTC ATT TTG ATT GGT ACT GGC TCA GAG          1979
Ser Ser Gly Asn Lys Pro Asp Val Ile Leu Ile Gly Thr Gly Ser Glu
625                 630                 635                 640
TTA GAA ATT GCT GTC AAG GCT GCT GAT GAA CTC AGG AAA GAA GGA AAA          2027
Leu Glu Ile Ala Val Lys Ala Ala Asp Glu Leu Arg Lys Glu Gly Lys
                645                 650                 655
GCA GTG AGA GTT GTT TCC TTT GTT TGT TGG GAG CTT TTT GAA GAA CAA          2075
Ala Val Arg Val Val Ser Phe Val Cys Trp Glu Leu Phe Glu Glu Gln
                660                 665                 670
TCA GCC GAC TAC AAG GAA AGT GTC CTT CCA TCA TCT GTT ACA GCT AGA          2123
Ser Ala Asp Tyr Lys Glu Ser Val Leu Pro Ser Ser Val Thr Ala Arg
                675                 680                 685
GTT AGC ATT GAG GCC GGA TCC ACA TTT GGG TGG GAG AAA TAT GTC GGA          2171
Val Ser Ile Glu Ala Gly Ser Thr Phe Gly Trp Glu Lys Tyr Val Gly
                690                 695                 700
TCA AAG GGG AAG GCC ATC GGA ATT GAC AGA TGG GGT GCC AGT GCC CCT          2219
Ser Lys Gly Lys Ala Ile Gly Ile Asp Arg Trp Gly Ala Ser Ala Pro
705                 710                 715                 720
```

```
GCT GGA AAA ATA TAC AAG GAG TAC GGA ATT ACA GCA GAG GCT GTT GTA          2267
Ala Gly Lys Ile Tyr Lys Glu Tyr Gly Ile Thr Ala Glu Ala Val Val
                725                 730                 735
GCT GCA GCT AAA CAA GTT TCT T AGGCTTTATT ACTTACCCTT GGTTGCTGGT           2319
Ala Ala Ala Lys Gln Val Ser
                740
GTCTACCAAA TTTGTTTTCA TTTTGAAACT GAGGTTGGAG ATAACGGTGG AAACCAATAC        2379
CAAACGGACT CGGCAGTTCA CTGTTGCCTG GTATTTTCAA TAAAAACTAT TTCTTCATCT        2439
GTCCTTTGTT TTCTTCAGTT TTAGTAGCGG AGCGGCCAAA ATGAATCCAA GATGAGGATA       2499
GAAATAGGAT TATGGATGCT CCTGACCATG TACACTTAAA ACATATCTGT GAGTTTTGTA        2559
ATTTTATTTG GTCGAGTGAT ACCAAGATCT CATTTTCAAT GGAAAAAAAA AAAAAAAAAA        2619
AAAAAAAAAA                                                              2629
```

(2)  INFORMATION ZU SEQ ID NO: 2:

    (i)  SEQUENZ CHARAKTERISTIKA:

        (A)  LÄNGE: 743 Aminosäuren

        (B)  ART: Aminosäure

        (D)  TOPOLOGIE: linear

    (ii) ART DES MOLEKÜLS: Protein

    (xi) SEQUENZBESCHREIBUNG: SEQ ID NO: 2:

```
Met Ala Ser Ser Ser Ser Leu Thr Leu Ser Gln Ala Ile Leu Ser Arg
 1               5                  10                  15
Ser Val Pro Arg His Gly Ser Ala Ser Ser Ser Gln Leu Ser Pro Ser
            20                  25                  30
Ser Leu Thr Phe Ser Gly Leu Lys Ser Asn Pro Asn Ile Thr Thr Ser
             35                  40                  45
Arg Arg Arg Thr Pro Ser Ser Ala Ala Ala Ala Ala Val Val Arg Ser
        50                  55                  60
Pro Ala Ile Arg Ala Ser Ala Ala Thr Glu Thr Ile Glu Lys Thr Glu
 65                  70                  75                  80
Thr Ala Leu Val Asp Lys Ser Val Asn Thr Ile Arg Phe Leu Ala Ile
                85                  90                  95
Asp Ala Val Glu Arg Gln Ile Arg Val Thr Arg Phe Ala Met Gly Cys
            100                 105                 110
Ala Pro Met Gly His Ile Leu Tyr Asp Glu Val Met Arg Tyr Asn Pro
            115                 120                 125
Lys Asn Pro Tyr Trp Phe Asn Arg Asp Arg Phe Val Leu Ser Ala Gly
        130                 135                 140
His Gly Cys Met Leu Gln Tyr Ala Leu Leu His Leu Ala Gly Tyr Asp
145                 150                 155                 160
Ala Val Arg Glu Glu Asp Leu Lys Ser Phe Arg Gln Trp Gly Thr Lys
                165                 170                 175
Thr Pro Gly His Pro Glu Asn Phe Glu Thr Pro Gly Val Glu Val Thr
                180                 185                 190
Thr Gly Pro Leu Gly Gln Gly Ile Ala Asn Ala Val Gly Leu Ala Leu
            195                 200                 205
```

12

```
Val Glu Lys His Leu Ala Ala Arg Phe Asn Lys Pro Asp Ala Glu Ile
    210                 215                 220
Val Asp His Tyr Thr Tyr Val Ile Leu Gly Asp Gly Cys Gln Met Glu
225                 230                 235                 240
Gly Ile Ser Gln Glu Ala Cys Ser Leu Ala Gly His Trp Gly Leu Gly
                245                 250                 255
Lys Leu Ile Ala Phe Tyr Asp Asp Asn His Ile Ser Ile Asp Gly Asp
                260                 265                 270
Thr Glu Ile Ala Phe Thr Glu Asp Val Gly Ala Arg Phe Glu Ala Leu
            275                 280                 285
Gly Trp His Val Ile Trp Val Lys Asn Gly Asn Thr Gly Tyr Asp Glu
            290                 295                 300
Ile Arg Ala Ala Ile Lys Glu Ala Lys Thr Val Thr Asp Lys Pro Thr
305                 310                 315                 320
Met Ile Lys Val Thr Thr Thr Ile Gly Phe Gly Ser Pro Asn Lys Ala
                325                 330                 335
Asn Ser Tyr Ser Val His Gly Ser Ala Leu Gly Ala Lys Glu Val Glu
                340                 345                 350
Ala Thr Arg Ser Asn Leu Gly Trp Pro Tyr Glu Pro Phe His Val Pro
            355                 360                 365
Glu Asp Val Lys Ser His Trp Ser Arg His Val Pro Glu Gly Ala Ala
    370                 375                 380
Leu Glu Ala Gly Trp Asn Thr Lys Phe Ala Glu Tyr Glu Lys Lys Tyr
385                 390                 395                 400
Pro Glu Glu Ala Ala Glu Leu Lys Ser Ile Thr Thr Gly Glu Leu Pro
                405                 410                 415
Ala Gly Trp Glu Lys Ala Leu Pro Thr Tyr Thr Pro Glu Ser Pro Ala
            420                 425                 430
Asp Ala Thr Arg Asn Leu Ser Gln Gln Asn Leu Asn Ala Leu Ala Lys
            435                 440                 445
Val Leu Pro Gly Phe Leu Gly Gly Ser Ala Asp Leu Ala Ser Ser Asn
    450                 455                 460
Met Thr Leu Met Lys Met Phe Gly Asp Phe Gln Lys Asn Thr Pro Glu
465                 470                 475                 480
Glu Arg Asn Leu Arg Phe Gly Val Arg Glu His Gly Met Gly Ala Ile
            485                 490                 495
Cys Asn Gly Asn Ala Leu His Ser Pro Gly Leu Ile Pro Tyr Cys Ala
            500                 505                 510
Thr Phe Phe Val Phe Thr Asp Tyr Met Arg Gly Ala Met Arg Ile Ser
            515                 520                 525
Ala Leu Ser Glu Ala Gly Val Ile Tyr Val Met Thr His Asp Ser Ile
    530                 535                 540
Gly Leu Gly Glu Asp Gly Pro Thr His Gln Pro Ile Glu His Leu Pro
545                 550                 555                 560
Ser Phe Arg Ala Met Pro Asn Ile Leu Met Phe Arg Pro Ala Asp Gly
                565                 570                 575
```

```
Lys Glu Thr Ala Gly Ala Tyr Lys Val Ala Val Leu Lys Arg Lys Thr
                580                 585                 590
Pro Ser Ile Leu Ala Leu Ser Arg Gln Lys Leu Pro Gln Leu Ala Gly
            595                 600                 605
Ser Ser Ile Glu Gly Ala Ala Lys Arg Gly Tyr Ile Leu Ser Asp Asn
        610                 615                 620
Ser Ser Gly Asn Lys Pro Asp Val Ile Leu Ile Gly Thr Gly Ser Glu
    625                 630                 635                 640
Leu Glu Ile Ala Val Lys Ala Ala Asp Glu Leu Arg Lys Glu Gly Lys
                645                 650                 655
Ala Val Arg Val Val Ser Phe Val Cys Trp Glu Leu Phe Glu Glu Gln
                660                 665                 670
Ser Ala Asp Tyr Lys Glu Ser Val Leu Pro Ser Ser Val Thr Ala Arg
            675                 680                 685
Val Ser Ile Glu Ala Gly Ser Thr Phe Gly Trp Glu Lys Tyr Val Gly
        690                 695                 700
Ser Lys Gly Lys Ala Ile Gly Ile Asp Arg Trp Gly Ala Ser Ala Pro
    705                 710                 715                 720
Ala Gly Lys Ile Tyr Lys Glu Tyr Gly Ile Thr Ala Glu Ala Val Val
                725                 730                 735
Ala Ala Ala Lys Gln Val Ser
                740
```